# EUROPEAN PATENT SPECIFICATION

(11) **EP 3 017 820 B1**
(45) Date of publication and mention of the grant of the patent: **26.06.2019**
(21) Application number: 14820283.1
(22) Date of filing: 03.07.2014
(51) Int. Cl.: A61K 35/64, A61K 31/593, A61P 31/12

(54) **APPLICATION OF ADSORBED DRONE BROOD HOMOGENATE AND OF VITAMIN D3 OR D-GROUP VITAMINS AND/OR ACTIVE METABOLITES THEREOF FOR PREVENTING AND TREATING VIRAL DISEASES**
VERWENDUNG VON ADSORBIERTEM DROHNENBRUT-HOMOGENAT UND VON VITAMIN D3 ODER VITAMINEN DER D-GRUPPE UND/ODER AKTIVEN METABOLITEN DAVON ZUR VORBEUGUNG UND BEHANDLUNG VON VIRUSERKRANKUNGEN
UTILISATION DE L'HOMOGÉNAT DE COUVAIN DE FAUX-BOURDONS ADSORBÉ ET DE VITAMINE D3 OU DE VITAMINES DU GROUPE D ET/OU DE LEURS MÉTABOLITES ACTIFS POUR LA PRÉVENTION ET LE TRAITEMENT DES MALADIES VIRALES

(30) Priority: 03.07.2013 RU 2013130302
(43) Date of publication of application: 11.05.2016
(73) Proprietor: Obshestvo S Ogranichennoj Otvetstvennostju "PARAFARM", Penza 440026 (RU)
(72) Inventor: STRUKOV, Villorij Ivanovich, g. Penza 440011 (RU); PROKHOROV, Mikhail Dmitrievich, g. Penza 440011 (RU); JONES-STRUKOVA, Olga, Fort Worth Texas, 76107 (US); TRIFONOV, Vjacheslav Nikolaevich, Zarechnyj Penza 442960 (RU); ELISTRATOVA, Julija Anatol'evna, g. Penza 440026 (RU); ELISTRATOV, Konstantin Gennad'evich, g. Penza 440061 (RU); KURUS', Natalya Vjacheslavovna, g. Penza 440026 (RU); EREMINA, Natalya Vyacheslavovna, g. Penza 440047 (RU); MAKSIMOVA, Marina Nikolaevna, g. Penza 440044 (RU); GALEEVA, Ramziya Timurshovna, g. Penza 440062 (RU); RADCHENKO, Larisa Grigorievna, g. Penza 440062 (RU); FEDOROV, Aleksandr Viktorovich, g. Penza 440026 (RU); KRUTYAKOV, Evgeny Nikolaevich, g. Penza 440011 (RU); ELISTRATOVA, Tatiana Viktorovna, g. Penza 440026 (RU); KHOMYAKOVA, Irina Vladimirovna, g. Penza 440026 (RU); TOLBINA, Galina Anatolievna, g. Penza 440026 (RU)
(74) Representative: Engel, Christoph Klaus
(86) International application number: PCT/RU2014/000487
(87) International publication number: WO 2015/002576

(56) References cited:
- WO-A1-2013/039424
- RU-C1- 2 233 666
- US-A- 5 580 297
- GERRY K. SCHWALFENBERG: "A review of the critical role of vitamin D in the functioning of the immune system and the clinical implications of vitamin D deficiency", MOLECULAR NUTRITION & FOOD RESEARCH, vol. 55, no. 1, 1 January 2011 (2011-01-01), pages 96-108, XP055017925, ISSN: 1613-4125, DOI: 10.1002/mnfr.201000174
- URASHIMA M ET AL.: 'Randomized trial of vitamin D supplementation to prevent seasonal influenza A in schoolchildren.' AM J CLIN NUTR. vol. 91, no. 5, 2010, pages 1255 - 1260, XP055310238
- TRUTNEVYI GOMOGENAT 18 January 2013, pages 1 - 3, XP055310241 Retrieved from the Internet: <URL:http://www.edka.ru/food/trutnev-homoge nate>

## Description

The invention relates to medicine, in particular, to agents for prophylaxis and treatment of conditions associated with various forms of catarrhal diseases, as well as for immunity improvement. The invention is defined in the claim.

It is known that adsorbed drone brood homogenate and vitamin D3 or other D group vitamins (and/or their active metabolites) are used for closing cavity formations in metaphyseal (trabecular) bone sections, and for preventing secretion of calcium therefrom, which provides the reduction and elimination of bone cavities, and prevents the formation thereof (patent RU 92466732, 31.05.2011).

Drone brood homogenate is used to prevent and treat catarrhal diseases such as flu (Trutnevy gomogenat, 18.1.2013. http://www.edka.ru/food/trutnev-homogenate, pages 1-3).

While conducting the studies of aforementioned "Osteo-vit D3" for the prophylaxis and treatment of bone tissue hypermineralization (during 2012- 2013), it was found that there were no cases of respiratory viral diseases, particularly, influenza, in patients who received this preparation. Moreover, it was found that general well-being and immunity improved in patients receiving the aforementioned preparation.

During these studies no other drug having similar composition has been found, which allows the achievement the mentioned effect.

Hence, it was found that a new technical result, namely, the prophylaxis and treatment of catarrhal and respiratory viral diseases, is achieved due to the administration of the known drug (adsorbed drone brood homogenate and vitamin D3 or other D group vitamins and/or their active metabolites).

This technical result is achieved due to daily intake of 10 mg to 1000 mg of adsorbed drone brood homogenate and 50 I.U. to 100,000 I.U. of vitamin D3 or D group vitamins (and/or their active metabolites).

The wide dose range of the claimed drug is explained by individual characteristics of patient: age, diet, lifestyle, race, country of residence, gender, inherited and past illnesses. By evaluating these criteria, doctor selects the individual doses of components of the claimed drug and makes corrections based on the dynamics of treatment.

### Example 1.

The study on the efficiency of the preparation was conducted in a group, consisting of 22 patients aged from 32 to 68 years, among them 9 were male, 13 - female. The patients received 1 tablet of the preparation 2 times a day during three-month course three times a year with monthly intervals by oral or sublingual administration in order to determine protective efficiency of the preparation against catarrhal and respiratory diseases.

As a result of the study, after 6-month therapy in the aforementioned group the immunity improvement was clinically observed, which was confirmed by objective examination data: there were no cases of acute respiratory disease or influenza in the patients during the autumn and winter period.

### Example 2.

Additional studies were conducted after the aforementioned studies. A group, consisting of 9 patients, aged from 21 to 59 years, 4 of them were male, 5 - female, with the initial stage of influenza received 1 tablet of the aforementioned preparation two times a day during 1 month via sublingual administration.

As a result, the acute stage with the expressed symptoms of viral respiratory disease didn't develop in the patients of the studied group: the temperature, headache, chills and runny nose were absent. There were no such complications as bronchitis.

### Example 3.

Studies were conducted in a group of 11 females aged from 37 to 64 years with ARVI diagnosis. The preparation was prescribed to the aforementioned group in a dose of 1 tablet two times a day, during one month via sublingual administration.

As a result, in the patients of this group the disease was in a milder form: the body temperature didn't increase over 38 degrees, severe nasal congestion and runny nose were absent. Moreover, the recovery period was by 2-5 days shorter that in patients, who didn't receive the preparation.

## Claims

1. A composition for use in the treatment and the prophylaxis of viral diseases, where the composition comprises
- 10 mg to 1000 mg of adsorbed drone brood homogenate and
- 50 IU to 100,000 IU of vitamin D or D group vitamins and/or their active metabolites,
wherein the composition is provided as a tablet.

## Patentansprüche

1. Eine Zusammensetzung zur Verwendung bei der Behandlung und Vorbeugung von Viruserkrankungen, wobei die Zusammensetzung beinhaltet
- 10 mg bis 1000 mg von adsorbiertem Drohnenbruthomogenat und
- 50 ME bis 100.000 ME von Vitamin D oder Vitaminen der D-Gruppe und/oder aktiven Metaboliten davon,
wobei die Zusammensetzung in Tablettenform bereitgestellt wird.

## Revendications

1. Composition destinée à être utilisée dans le traitement et la prophylaxie de maladies virales, où la composition comprend
- 10 mg à 1000 mg d'homogénat de couvain de faux bourdons adsorbé et
- 50 UI à 100000 UI de vitamine D ou de vitamines du groupe D
et/ou leurs métabolites actifs, où la composition est fournie sous forme de comprimé.
